(19) Europäisches Patentamt

European Patent Office

Office européen des brevets

(11) **EP 1 327 437 B1**

(12) **FASCICULE DE BREVET EUROPEEN**

(45) Date de publication et mention
de la délivrance du brevet:
**17.05.2006 Bulletin 2006/20**

(51) Int Cl.:
*A61K 8/97* (2006.01)     *A61K 8/49* (2006.01)
*A61Q 19/04* (2006.01)

(21) Numéro de dépôt: **02292986.3**

(22) Date de dépôt: **03.12.2002**

(54) **Compositions pour la coloration de la peau comprenant un extrait de sorgho et utilisation**

Hautfärbemittel enthaltend ein Sorghumextrakt und Verwendung davon

Skin dyeing compositions containing a sorghum extract and use

(84) Etats contractants désignés:
**AT BE BG CH CY CZ DE DK EE ES FI FR GB GR IE IT LI LU MC NL PT SE SI SK TR**

(30) Priorité: **10.01.2002 FR 0200251**
**10.01.2002 FR 0200252**
**10.01.2002 FR 0200253**

(43) Date de publication de la demande:
**16.07.2003 Bulletin 2003/29**

(73) Titulaire: **L'OREAL**
**75008 Paris (FR)**

(72) Inventeurs:
• **Seyler, Nathalie**
**94700 Maisons-Alfort (FR)**
• **Elguidj, Irène**
**92200 Neuilly (FR)**
• **Candau, Didier**
**91570 Bievres (FR)**

(74) Mandataire: **Miszputen, Laurent**
**L'OREAL - D.I.P.I.**
**25-29 Quai Aulagnier**
**92600 Asnières (FR)**

(56) Documents cités:
**WO-A-02/41867**      **FR-A- 2 757 383**

• **DATABASE WPI Week 199929 Derwent Publications Ltd., London, GB; AN 1999-338248 XP002214396 & CN 1 209 992 A (ZHANG)**
• **DATABASE WPI Week 198828 Derwent Publications Ltd., London, GB; AN 1988-195805 XP002214397 & JP 63 135310 A (LION CORP.)**
• **DATABASE BIOSIS [en ligne] BIOSCIENCES INFORMATION SERVICE, PHILADELPHIA, PA, US; 1993 REY J -P ET AL: "Isolation and composition of a natural dye from the stems of Sorghum bicolor (L.) Moench subsp. americanum caudatum." Database accession no. PREV199497073236 XP002214398 & CEREAL CHEMISTRY, vol. 70, no. 6, 1993, pages 759-760, ISSN: 0009-0352**

EP 1 327 437 B1

**Description**

La présente invention se rapporte à des compositions cosmétiques et/ou dermatologiques destinées à la coloration artificielle de la peau caractérisée par le fait qu'elle comprend, dans un support cosmétiquement acceptable, au moins un extrait de sorgho ; ladite composition ne contenant pas de sel de flavylium non substitué en position 3 et substitué par au moins un radical hydroxyle ou alcoxy.

L'invention concerne également les applications de ces compositions à la coloration de la peau proche du bronzage naturel de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Il existe donc une demande croissante de produits autobronzants agissant rapidement et conférant une coloration plus proche du bronzage naturel.

Ainsi, on est toujours, à la recherche de nouveaux composés et de nouvelles compositions permettant de conférer artificiellement à la peau une coloration proche du bronzage naturel d'une manière simple, efficace, rapide et sans risque.

Les extraits de sorgho sont connus depuis longtemps comme colorants alimentaires. Ils procurent une teinte marron-rouge et présentent dans leur composition des flavonoïdes, des anthocyanidines et des tannins.

Or, à la suite d'importantes recherches menées dans le domaine de la coloration artificielle de la peau, la Demanderesse a maintenant découvert que l'utilisation à titre d'agent de coloration de la peau, d'un extrait de sorgho, en l'absence de sel de flavylium non substitué en position 3 et substitué par au moins un radical hydroxyle ou alcoxy, permettait de conférer immédiatement après l'application du produit sur la peau une coloration artificielle proche du bronzage naturel.

La présente invention a donc pour objet une nouvelle composition cosmétique dermatologique, destinée à la coloration artificielle de la peau proche du bronzage naturel, caractérisée par le fait qu'elle comprend, dans un support cosmétiquement acceptable, au moins un extrait de sorgho ; ladite composition ne contenant pas de sel de flavylium non substitué en position 3 et substitué par au moins un radical hydroxyle ou alcoxy.

La présente invention a encore pour objet l'utilisation nouvelle d'au moins un extrait de sorgho, dans une composition cosmétique ne contenant pas de sel de flavylium non substitué en position 3 et substitué par au moins un radical hydroxyle ou alcoxy, dans le but de conférer à la peau une coloration artificielle proche du bronzage naturel.

La présente invention a également pour objet un procédé de coloration artificielle proche du bronzage naturel de la peau, caractérisé en ce qu'il consiste à appliquer sur celle-ci une quantité efficace d'une composition cosmétique telle que définie précédemment.

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

Au sens de la présente invention, on entendra, par « composition destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

D'autres caractéristiques, aspects et avantages de la présente invention apparaîtront à la lecture de la description détaillée qui va suivre.

Les compositions conformes à la présente invention conduisent en général au bout de 30 minutes après application sur une peau claire à raison de 2mg/cm$^2$ à un assombrissement caractérisé dans le système de mesure colorimétrique (L*, a*, b*) par un ΔL* allant de -0,5 à - 20. De façon plus préférentielle, ΔL* variera de - 0,5 à -15.

Les compositions conformes à la présente invention apportent au bout de 30 minutes après application sur la peau à raison de 2mg/cm² une coloration sur une peau claire qui est définie dans le système de mesure colorimétrique (L*, a*, b*), par un rapport Δa*/Δb* allant de 0,5 à 3 et encore plus particulièrement de 0, 8 à 2.

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au 16th IFSCC congress oct 8-10 1990 de New-York, et in *Int. J. Cosm. Sci.* **13** 191-208 (1991). Les peaux claires telles que définies dans cette classification ont un angle ITA compris entre 35 et 55.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

ΔL* traduit l'assombrissement de la couleur : plus le ΔL* est négatif, plus la couleur s'est assombrie avec :

$$\Delta L^* = L^* \text{ peau non colorée} - L^* \text{ peau colorée}$$

Le rapport Δa*/Δb* traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Les extraits de sorgho conformes à l'invention sont obtenus à partir de la plante entière, des tiges, des graines ou des feuilles du genre Sorghum. Les espèces préférées du Sorghum sont choisies parmi le Sorghum bicolor, le Sorghum caudatum, le Sorghum nervosum, le Sorghum durra, le Sorghum vulgare et les Sorghum en association avec du Colletotrichum Graminicola.

Les extraits de sorgho conformes à l'invention sont plus particulièrement les extraits de *Sorghum vulgare* tels que le produit commercial Sorghum Extract Absorbance >30 vendu par la société Premier Specialties .

Les extraits de sorgho conformes à l'invention sont issus de l'extraction de la plante entière ou des parties de plantes citées ci-dessus qui peuvent être à l'état frais ou à l'état sec.

Les extraits de sorgho conformes à l'invention peuvent être obtenus selon un procédé comprenant les étapes suivantes :

(a) une extraction de la plante entière, des tiges, des graines ou des feuilles du genre Sorghum dans un milieu aqueux pouvant contenir en plus au moins un solvant organique ;
(b) une macération dans un milieu alcalin présentant un pH de l'ordre 11-12.
(c) éventuellement une précipitation du milieu de macération par addition d'un acide de telle sorte à atteindre un pH de l'ordre de 1-2.

L"extraction peut être réalisée en milieu acide comme décrit dans les brevets chinois CN 1035512 C et CN 1064284A et dans la publication de M KOUDA-BONAFOS, E CZYZEWSKA, M NACRO et AC OEHLSCHLAGER. « Isolation of apigenin from leaf sheats of Sorghum caudatum » Journal of Chemical ecology, Vol. 20, N°8, p2123-2125 (1995).

Elle peut également être réalisée en milieu alcalin suivi d'une précipitation en milieu acide comme décrit dans le brevet chinois CN 1065079A et dans la publication de JP REY, JL POUSSET, J LEVESQUE et P WANTY. « isolation and composition of a natural dye from the stems of sorghum bicolor (L.) Moench subsp. Americanum caudatum » Cereal Chem. Vol 70(6), p759-760 (1993).

L'extraction peut aussi être réalisée en milieu organique comme décrit dans les publications de J WANG « Studies on extraction of pigment from sorghum husks and its properties » Huaxue Shijie, Vol 39 (4), p211-213 (1998) et de A SEREME, M KOUDA-BONAFOS et M NACRO « Phenolic compounds in Sorghum caudatum tissues during plant development » Biomass and Bioenergy Vol 4(1), p69-71 (1993).

Les solvants organiques utilisés pour l'extraction peuvent être des alcools comme l'éthanol, le méthanol, l'alcool propylique normal primaire, l'alcool isopropylique, l'alcool butylique normal primaire, le propylène glycol et le glycérol par exemple. Les solvants organiques peuvent être également représenté par l'éther diéthylique, l'acétone, l'éthylméthylcétone, l'acétate d'éthyle par exemple. Les solvants organiques utilisés peuvent également être des fluides supercritiques ou des solvants fluorées comme le dodécafluoropentane, le tétradécafluorohexane, la N-méthyl morpholine perfluoré et le méthoxy nonafluorobutane par exemple.

L'étape (b) de macération en milieu alcalin peut être réalisée pendant une période de 15-25 jours à une température de 60-80°C dans une solution d'hydroxyde de sodium 0.1N présentant un pH de l'ordre de 11-12.

L'étape (c) de précipitation par addition d'acide peut être effectuée par exemple avec de l'acide chlorhydrique 10N pour atteindre un pH de l'ordre de 1-2. La suspension aqueuse ainsi obtenue est ensuite filtrée pour récupérer le précipité qui est ensuite séché.

Les étapes (b) et (c) peuvent être répétées plusieurs fois.

La concentration en extrait de Sorgho conforme à l'invention varie de préférence de 0,0001 à 10%, et encore plus préférentiellement de 0,001 à 5% en poids, par rapport au poids total de la composition.

Selon une forme particulière, les compositions de l'invention peuvent contenir en plus un ou plusieurs agents autobronzants mono ou polycarbonylés.

Les agents autobronzants mono ou polycarbonylés sont choisis par exemple parmi l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrits dans la demande de brevet FR 2466492 et WO 9735842, la dihydroxyacétone (DHA), les dérivés de 4,4-dihydroxypyrazolin-5-ones telles que décrits dans la demande de brevet EP 903342, ces agents autobronzants pouvant être associés ou non à des colorants directs ou des dérivés indoliques.

Dans un mode de réalisation préféré de l'invention on utilisera plus particulièrement la dihydroxyacétone (DHA).

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Selon une forme particulière, les compositions de l'invention peuvent contenir en plus un ou plusieurs agents filtrant le rayonnement ultra violet.

Les agents filtrant le rayonnement ultra violet peuvent être choisis parmi les filtres UV organiques ou les agents filtrant les radiations UV minéraux.

Les filtres UV organiques conformes à l'invention peuvent être hydrosolubles, liposolubles ou insolubles dans les solvants usuels cosmétiques. Ils sont choisis notamment parmi les anthranilates; les dérivés cinnamiques; les dérivés de dibenzoylméthane; les dérivés salicyliques, les dérivés du camphre; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de benzalmalonate, les dérivés de $\beta,\beta'$-diphénylacrylate, les dérivés de benzotriazole, les dérivés de benzimidazole ; les imadazolines; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93104665 ; les dimères dérivés d'$\alpha$-alkylstyrène tels que ceux décrits dans la demande de brevet DE19855649 ; les dérivés de 4,4-diarylbutadiène tels que ceux décrits dans les demandes de brevet EP0967200, DE19755649, EP1333981 et leurs mélanges.

Comme exemples de filtres organiques, on peut citer ceux désignés ci-dessous sous leur nom INCI:

Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

Dérivés salicyliques :

- Homosalate vendu sous le nom « EUSOLEX HMS » par RONA/EM INDUSTRIES,

- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques:

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate, .
- DEA Methoxycinnamate,
- -Diisopropyl Methylcinnamate,
- Glyceryl Ethylhexanoate Dimethoxycinnamate

Dérivés de β,β'-diphénylacryate:

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

Dérivés du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK ,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés du benzalmalonate :

- Polyorganosiloxane à fonctions benzalmalonate vendu sous la dénomination commerciale « PARSOL SLX » par HOFFMANN LA ROCHE

Dérivés de 4,4-diarylbutadiène :

- 1,1-dicarboxy (2,2'-diméthyl-propyl)-4,4-diphénylbutadiène et leurs mélanges.

Les filtres UV organiques plus particulièrement préférés sont choisis parmi les composés suivants :

- Ethylhexyl Salicylate,
- Butyl Methoxydibenzoylmethane,
- Ethylhexyl Methoxycinnamate,
- Octocrylene,
- Phenylbenzimidazole Sulfonic Acid,

- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol, 1, 1-dicarboxy (2,2'-diméthyl-propyl)-4,4-diphénylbutadiène
- Drometrizole Trisiloxane,

et leurs mélanges.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou ànatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les agents filtrants les radiations conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents répulsifs contre les insectes, les agents anti-mousse, les agents hydratants, les vitamines, les parfums, les conservateurs, les tensioactifs, les charges, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine) ; végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'utilisation des composés du type sel de flavylium conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Des exemples concrets, mais nullement limitatifs, illustrant l'invention, vont maintenant être donnés.

### EXEMPLE 1 :

Cet exemple vise à montrer, dans un premier temps, l'intensité de la coloration obtenue avec un extrait de sorgho conforme à la présente invention ainsi que la rapidité avec laquelle cette coloration se développe par rapport à une composition contenant la DHA à titre d'agent de coloration de la peau.

Cet exemple vise à montrer, dans un deuxième temps, que la coloration obtenue avec un extrait de sorgho conforme à la présente invention est proche de celle du bronzage naturel de la peau.

La Demanderesse a réalisé les compositions suivantes (les quantités sont exprimées en pourcentage de poids par rapport au poids total de la composition):

Composition $A_1$ (hors invention):

- Polydiméthyl/méthyl siloxane POE/POP(396/4) (OE/OP 18/18) A 10 % D5          10g
- Cyclopentadiméthylsiloxane          12.5g
- Mélange tocophérols naturels/huile de soja          0.1g
- Dihydroxyacétone (DHA)          4g
- Propylène glycol          15g
- Conservateurs          qs
- Eau déminéralisée          qsp100g

Composition $B_1$ (invention)

- Polydiméthyl/méthyl siloxane POE/POP(396/4) (OE/OP 18/18) A 10 % D5          10g
- Cyclopentadiméthylsiloxane          12.5g
- Mélange tocophérols naturels/huile de soja          0.1g
- Propylène glycol          15g
- Extrait de Sorghum vulgare
  (Sorghum Extract Absorbance >30 de Premier Specialties)          0.7g
- Conservateurs          qs
- Eau déminéralisée          qsp100g

### Protocole d'évaluation :

Les compositions $A_1$ et $B_1$ ont été appliquées à raison de 2 mg/cm$^2$ sur une zone de 2 * 2 cm$^2$ délimitée sur le dos de l'avant bras dont la couleur de peau caractérisée par l'angle ITA est compris entre 35 et 55..

Les séries de mesures colorimétriques suivantes ont été effectuées à l'aide d'un spectrocolorimètre Minolta CM-508d :

- 1°) avant application de la composition,
- 2°) 30 minutes après l'application,

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ peau non colorée} - L^* \text{ peau colorée}$$

Pour la nuance de la coloration obtenue, on s'intéresse au rapport $\Delta a^*/\Delta b^*$ qui traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

**Tableau (I) :**

| | Composition A$_1$ (comparative) $\Delta L^*$ | Composition B$_1$ (invention) $\Delta L^*$ | Composition B$_1$ (invention) $\Delta a^*/\Delta b^*$ |
|---|---|---|---|
| T = 30 minutes | -0.4 | -5.6 | 2.7 |

On constate ainsi que 30 minutes après l'application, la composition B$_1$ contenant la DHA et l'extrait végétal colorant permet d'obtenir un assombrissement beaucoup plus intense que celui obtenu avec la composition A$_1$ contenant uniquement la DHA.

La composition B$_1$ contenant la DHA et l'extrait végétal colorant permet également d'obtenir une nuance proche du bronzage naturel .

**Exemple 2**

On procède dans les mêmes conditions que l'exemple 1.

Composition A$_2$ (hors invention):

- Polydiméthyl/méthyl siloxane POE/POP(396/4) (OE/OP 18/18) A 10 % D5        10g
- Cyclopentadiméthylsiloxane          12.5g
- Mélange tocophérols naturels/huile de soja          0.1g
- Dihydroxyacétone (DHA)        4g
- Propylène glycol          15g
- Conservateurs          qs
- Eau déminéralisée          qsp 100g

Composition B$_2$ (invention)

- Polydiméthyl/méthyl siloxane POE/POP(396/4) (OE/OP 18/18) A 10 % D5        10g
- Cyclopentadiméthylsiloxane          12.5g
- Mélange tocophérols naturels/huile de soja          0.1g
- Propylène glycol          15g
- Extrait de Sorghum vulgare
  (Sorghum Extract Absorbance >30 de Premier Specialties)          0.7g
- Dihydroxyacétone        4g
- Conservateurs          qs
- Eau déminéralisée          qsp 100g

Les résultats obtenus sont rassemblés dans le tableau (II) suivant :

Tableau (II) :

| | Composition A₂ (comparative) ΔL* | Composition B₂ (invention) ΔL* | Composition B₂ (invention) Δa*/Δb* |
|---|---|---|---|
| T = 30 minutes | -0.4 | -5 | 1.2 |

On constate ainsi que 30 minutes après l'application, la composition A₂, qui contient à titre d'agent de coloration de la peau, uniquement la DHA, n'a conféré qu'une très faible coloration à la peau, puisque la DHA n'a pas encore eu le temps d'agir ($\Delta L^* = -0,4$). En revanche, la composition B₂ selon l'invention contenant la DHA associée à l'extrait de Sorgho a déjà conféré à la peau une coloration significative ($\Delta L^*=-5$).

La composition A₂ ne permet pas d'obtenir au bout de 30 minutes un assombrissement comparable à celui de la composition B₂.

## Exemple 3

On procède dans les mêmes conditions que l'exemple 1.

Composition A₃ (hors invention):

- Polydiméthyl/méthyl siloxane POE/POP(396/4) (OE/OP 18/18) A 10 % D5          10g
- Cyclopentadiméthylsiloxane          12.5g
- Mélange tocophérols naturels/huile de soja          0.1g
- Dihydroxyacétone (DHA)          4g
- Propylène glycol          15g
- Conservateurs          qs
- Eau déminéralisée          qsp 100g

Composition B₃ (invention)

- Polydiméthyl/méthyl siloxane POE/POP(396/4) (OE/OP 18/18) A 10 % D5          10g
- Cyclopentadiméthylsiloxane          12.5g
- Mélange tocophérols naturels/huile de soja          0.1g
- Acide benzène 1,4-di(3-méthylidène-10-camphosulfonique)          0,5g
- Propylène glycol          15g
- Extrait de Sorghum vulgare
  (Sorghum Extract Absorbance >30 de Premier Specialties)          0.7g
- Conservateurs          qs
- Eau déminéralisée          qsp 100g

Les résultats obtenus sont rassemblés dans le tableau (III) suivant :

Tableau (III) :

| | Composition A₃ (comparative) ΔL* | Composition B₃ (invention) ΔL* |
|---|---|---|
| T = 30 minutes | -0.4 | -6.1 |

On constate ainsi que 30 minutes après l'application, la composition A₃, qui contient, à titre d'agent de coloration de la peau, la DHA, n'a conféré qu'une très faible coloration à la peau, puisque la DHA n'a pas encore eu le temps d'agir ($\Delta L^* = -0.4$). En revanche, la composition B₃ selon l'invention contenant l'extrait de Sorgho associé à un filtre UV a déjà conféré à la peau une coloration significative ($\Delta L^*=-6.1$).

**Revendications**

1. Composition cosmétique et/ou dermatologique pour la coloration artificielle de la peau proche du bronzage naturel, **caractérisée par le fait qu'**elle comprend, dans un support cosmétiquement acceptable, au moins un extrait de sorgho ; ladite composition ne contenant pas de sel de flavylium non substitué en position 3 et substitué par au moins un radical hydroxyle ou alcoxy.

2. Composition selon la revendication 1, selon laquelle ledit extrait de sorgho est présent dans une quantité efficace permettant d'obtenir au bout de 30 minutes après application sur une peau claire à raison de 2mg/cm2 un assombrissement **caractérisé** dans le système de mesure colorimétrique L* a* b* par un ΔL* allant de - 0,5 à -20.

3. Composition selon la revendication 2, où le ΔL* varie de - 0,5 à -15 .

4. Composition selon l'une quelconque des revendications 1 à 3, selon laquelle ledit extrait de sorgho est présent dans une quantité efficace permettant d'obtenir au bout de 30 minutes après application sur une peau claire à raison de 2mg/cm$^2$ une coloration définie dans le système colorimétrique L* a* b* par un rapport Δa*/Δb* allant de 0,5 à 3.

5. Composition selon la revendication 4, où le rapport Δa*/Δb* varie de 0,8 à 2.

6. Composition selon l'une quelconque des revendications 1 à 5 où ledit extrait de sorgho est obtenu à partir de la plante entière, de tiges, de graines ou de feuilles du genre Sorghum, à l'état frais ou sec.

7. Composition selon la revendication 6 où les espèces du Sorghum sont choisies parmi le Sorghum bicolor, de Sorghum caudatum, de Sorghum nervosum, de Sorghum durra, de Sorghum vulgare ou les Sorghum en association avec du Colletotrichum Graminicola.

8. Composition selon l'une quelconque des revendications 1 à 7 où ledit extrait de sorgho est obtenu à partir de la plante entière, de tiges, de graines ou de feuilles de Sorghum vulgare.

9. Composition selon l'une quelconque des revendications 1 à 8 où l'extrait de sorgho est susceptible d'être obtenu selon un procédé comprenant les étapes suivantes :

   (a) une extraction de la plante entière, de tiges, de graines ou de feuilles de Sorghum dans un milieu aqueux pouvant contenir en plus au moins un solvant organique ;
   (b) une macération dans un milieu alcalin présentant un pH de l'ordre 11-12.
   (c) éventuellement une précipitation du milieu de macération par addition d'un acide de telle sorte à atteindre un pH de l'ordre de 1-2.

10. Composition selon l'une quelconque des revendications 1 à 9, où la concentration en extrait de Sorgho varie 0,0001 à 10%, et préférentiellement de 0,001 à 5% en poids, par rapport au poids total de la composition.

11. Composition selon l'une quelconque des revendications 1 à 10, contenant en plus au moins un autobronzant mono ou polycarbonylé.

12. Composition selon la revendication 11, où le ou les autobronzants mono ou polycarbonylés sont choisis parmi l'isatine, l'alloxane, la ninhydrine, le glycéraldéhyde, l'aldéhyde mésotartrique, la glutaraldéhyde, l'érythrulose, les dérivés de pyrazoline-4,5-diones, la dihydroxyacétone (DHA), les dérivés de 4,4-dihydroxypyrazolin-5-ones ; ces autobronzants pouvant être associés ou non à des colorants directs ou des dérivés indoliques.

13. Composition selon la revendication 11 ou 12, où l'autobronzant est la dihydroxyacétone (DHA).

14. Composition selon l'une quelconque des revendications 11 à 13, où l'autobronzant est présent dans des proportions allant de 0,1 à 10% en poids par rapport au poids total de la composition.

15. Composition selon l'une quelconque des revendications 1 à 14, contenant en plus au moins un agent filtrant les radiations UV.

16. Composition selon la revendication 15, où ledit agent filtrant les radiations UV est choisi parmi les filtres UV organiques ou les agents filtrant les radiations UV minéraux.

**17.** Composition selon la revendication 16, où le ou les filtres UV organiques sont hydrosolubles, liposolubles ou insolubles dans les solvants usuels cosmétiques.

**18.** Composition selon la revendication 17, où le ou les filtres UV organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate, les dérivés de benzotriazole, les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les dérivés de 4,4-diarylbutadiène et leurs mélanges.

**19.** Composition selon la revendication 12, où le ou les agents filtrants les radiations UV organiques sont choisis parmi :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- Drometrizole Trisiloxane,
- 1,1-dicarboxy (2,2'-diméthyl-propyl)-4,4-diphénylbutadiène

et leurs mélanges.

**20.** Composition selon la revendication 16, où le ou les agents filtrant les radiations UV minéraux sont choisis parmi les pigments ou les nanopigments d'oxydes métalliques enrobés ou non.

**21.** Composition selon la revendication 20, où le ou les agents filtrant les radiations UV minéraux sont choisis parmi les nanopigments d'oxyde de titane, de fer, de zinc, de zirconium ou de cérium, enrobés ou non.

**22.** Composition selon l'une quelconque des revendications 15 à 21, où le ou les agents filtrant les radiations UV sont présents dans des proportions allant de 0,1 à 15% en poids par rapport au poids total de la composition.

**23.** Procédé de coloration artificielle proche du bronzage naturel de la peau, **caractérisé en ce qu'**il consiste à appliquer sur celle-ci une quantité efficace d'une composition telle que définie à l'une quelconque des revendications 1 à 10.

**24.** Utilisation d'au moins un extrait de sorgho tel que défini à l'une quelconque des revendications précédentes dans une composition cosmétique ne contenant pas de sel de flavylium non substitué en position 3 et substitué par au moins un radical hydroxyle ou alcoxy, dans le but de conférer à la peau une coloration artificielle proche du bronzage naturel.

**Claims**

**1.** Cosmetic composition for artificially colouring the skin close to the coloration of a natural tan, **characterized in that** it comprises, in a cosmetically acceptable support, at least one sorghum extract, the said composition not containing any flavylium salt unsubstituted in position 3 and substituted with at least one hydroxyl or alkoxy radical.

2. Composition according to Claim 1, according to which the said sorghum extract is present in an amount effective to produce, 30 minutes after applying to fair skin at a rate of 2 mg/cm$^2$, darkening **characterized in** the L*a*b* colorimetric measuring system by a $\Delta$L* ranging from -0.5 to -20.

3. Composition according to Claim 2, in which the $\Delta$L* ranges from -0.5 to -15.

4. Composition according to any one of Claims 1 to 3, according to which the said sorghum extract is present in an amount effective to produce, 30 minutes after applying to fair skin at a rate of 2 mg/cm$^2$, a coloration defined in the L*a*b* colorimetric system by a ratio $\Delta$a*/$\Delta$b* ranging from 0.5 to 3.

5. Composition according to Claim 4, in which the ratio $\Delta$a*/$\Delta$b* ranges from 0.8 to 2.

6. Composition according to any one of Claims 1 to 5, in which the said sorghum extract is obtained from the whole plant, stalks, seeds or leaves of the genus Sorghum, in fresh or dry form.

7. Composition according to Claim 6, in which the Sorghum species are chosen from Sorghum bicolor, Sorghum caudatum, Sorghum nervosum, Sorghum durra, Sorghum vulgare or Sorghums in combination with Colletotrichum graminicola.

8. Composition according to any one of Claims 1 to 7, in which the said sorghum extract is obtained from the whole plant, stalks, seeds or leaves of Sorghum vulgare.

9. Composition according to any one of Claims 1 to 8, in which the sorghum extract may be obtained according to a process comprising the following steps:

   (a) extraction of the whole Sorghum plant, stalks, seeds or leaves in an aqueous medium that may also contain at least one organic solvent;
   (b) maceration in an alkaline medium with a pH of about 11-12;
   (c) optionally, precipitation of the maceration medium by addition of an acid so as to reach a pH of about 1-2.

10. Composition according to any one of Claims 1 to 9, in which the concentration of Sorghum extract ranges from 0.0001% to 10% and preferentially from 0.001% to 5% by weight relative to the total weight of the composition.

11. Composition according to any one of Claims 1 to 10, also containing at least one monocarbonyl or polycarbonyl self-tanning agent.

12. Composition according to Claim 11, in which the monocarbonyl or polycarbonyl self-tanning agent(s) is (are) chosen from isatin, alloxan, ninhydrin, glyceraldehyde, mesotartaric aldehyde, glutaraldehyde, erythrulose, pyrazoline-4,5-dione derivatives, dihydroxyacetone (DHA) and 4,4-dihydroxypyrazolin-5-one derivatives, these self-tanning agents possibly being combined with direct dyes or indole derivatives.

13. Composition according to Claim 11 or 12, in which the self-tanning agent is dihydroxyacetone (DHA).

14. Composition according to any one of Claims 11 to 13, in which the self-tanning agent is present in proportions ranging from 0.1% to 10% by weight relative to the total weight of the composition.

15. Composition according to any one of Claims 1 to 14, also containing at least one UV-radiation screening agent.

16. Composition according to Claim 15, in which the said UV-radiation screening agent is chosen from organic UV-screening agents and mineral UV-radiation screening agents.

17. Composition according to Claim 16, in which the organic UV-screening agent(s) is (are) water-soluble, liposoluble or insoluble in the usual cosmetic solvents.

18. Composition according to Claim 17, in which the organic UV-screening agent(s) is (are) chosen from anthranilates; cinnamic derivatives; dibenzoylmethane derivatives; salicylic derivatives; camphor derivatives; triazine derivatives; benzophenone derivatives; $\beta$,$\beta$'-diphenylacrylate derivatives; benzotriazole derivatives; benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; α-alkylstyrene-based dimers; 4,4-diarylbutadiene derivatives, and mixtures thereof.

19. Composition according to Claim 12, in which the organic UV-radiation screening agent(s) is (are) chosen from:

- Ethylhexyl salicylate,
- Butylmethoxydibenzoylmethane,
- Ethylhexyl methoxycinnamate,
- Octocrylene,
- Phenylbenzimidazolesulfonic acid,
- Terephthalylidenedicamphorsulfonic acid,
- Benzophenone-3,
- Benzophenone-4,
- Benzophenone-5,
- 4-Methylbenzylidenecamphor,
- Benzimidazilate,
- Anisotriazine,
- Ethylhexyl triazone,
- Diethylhexyl butamido triazone,
- 2,4,6-Tris(diisobutyl 4'-aminobenzalmalonate)-s-triazine,
- Methylenebis(benzotriazolyl)tetramethylbutylphenol,
- Drometrizole trisiloxane,
- 1,1-Dicarboxy(2,2'-dimethylpropyl)-4,4-diphenylbutadiene,

and mixtures thereof.

20. Composition according to Claim 16, in which the mineral UV-radiation screening agent(s) is (are) chosen from coated or uncoated metal oxide pigments or nanopigments.

21. Composition according to Claim 20, in which the mineral UV-radiation screening agent(s) is (are) chosen from coated or uncoated titanium oxide, iron oxide, zinc oxide, zirconium oxide and cerium oxide nanopigments.

22. Composition according to any one of Claims 15 to 21, in which the UV-radiation screening agent(s) is (are) present in proportions ranging from 0.1% to 15% by weight relative to the total weight of the composition.

23. Process for artificially colouring the skin close to the coloration of a natural tan, **characterized in that** it consists in applying to the skin an effective amount of a composition as defined in any one of Claims 1 to 10.

24. Use of at least one sorghum extract as defined in any one of the preceding claims in a cosmetic composition not containing any flavylium salt unsubstituted in position 3 and substituted with at least one hydroxyl or alkoxy radical, for the purpose of giving the skin an artificial coloration close to that of a natural tan.


**Patentansprüche**

1. Kosmetische Zusammensetzung zur künstlichen Färbung der Haut in einer Farbnuance, die der natürlichen Bräunung nahe kommt, **dadurch gekennzeichnet, dass** sie in einem kosmetisch akzeptablen Träger mindestens einen Sorghum-Extrakt enthält; wobei die Zusammensetzung kein in 3-Stellung unsubstituiertes und mit mindestens einer Hydroxy- oder Alkoxygruppe substituiertes Flavyliumsalz enthält.

2. Zusammensetzung nach Anspruch 1, **dadurch gekennzeichnet, dass** der Sorghum-Extrakt in einer solchen Menge enthalten ist, dass 30 Minuten nach dem Auftragen in einer Menge von 2 mg/cm$^2$ auf helle Haut ein Dunklerwerden der Hautfarbe bewirkt wird, das im colorimetrischen L* a* b*-System durch ein ΔL* von -0,5 bis -20 **gekennzeichnet** ist.

3. Zusammensetzung nach Anspruch 2, wobei ΔL* im Bereich von -0,5 bis -15 liegt.

4. Zusammensetzung nach einem der Ansprüche 1 bis 3, wobei der Sorghum-Extrakt in einer solchen Menge enthalten ist, dass 30 Minuten nach dem Auftragen auf einen hellen Hauttyp in einer Menge von 2 mg/cm² eine Färbung erhalten wird, die im colorimetrischen System L\*, a\*, b\* durch ein Verhältnis Δa\*/Δb\* von 0,5 bis 3 definiert ist.

5.  Zusammensetzung nach Anspruch 4, wobei das Verhältnis Δa\*/Δb\* im Bereich von 0,8 bis 2 liegt.

6.  Zusammensetzung nach einem der Ansprüche 1 bis 5, wobei der Sorghum-Extrakt aus der ganzen Pflanze, den Stängeln, Samen oder Blättern von Pflanzen der Gattung Sorghum im frischen oder trockenen Zustand gewonnen wird.

7.  Zusammensetzung nach Anspruch 6, wobei die Sorghum-Arten unter *Sorghum bicolor, Sorghum caudatum, Sorghum nervosum, Sorghum durra, Sorghum vulgare* oder Sorghum in Kombination mit Colletotrichum Graminicola ausgewählt sind.

8.  Zusammensetzung nach einem der Ansprüche 1 bis 7, wobei der Sorghum-Extrakt aus der ganzen Pflanze, den Stängeln, Samen oder Blättern von Sorghum vulgare gewonnen wird.

9.  Zusammensetzung nach einem der Ansprüche 1 bis 8, wobei der Sorghum-Extrakt nach einem Verfahren erhältlich ist, das die folgenden Schritte umfasst:

    (a) Extraktion von Teilen aus der gesamten Pflanze, den Stängeln, den Samen oder den Blättern von Sorghum in einem wässrigen Medium, das ferner mindestens ein organisches Lösungsmittel enthalten kann;
    (b) Mazeration in einem alkalischen Medium, das einen pH-Wert in der Größenordnung von 11 bis 12 aufweist;
    (c) gegebenenfalls Fällen des Mazerationsmediums durch Zusatz einer Säure, die so zugegeben wird, dass der pH-Wert in der Größenordnung von 1 bis 2 eingestellt wird.

10. Zusammensetzung nach einem der Ansprüche 1 bis 9, wobei die Konzentration des Sorghum-Extrakts im Bereich von 0,0001 bis 10 % und vorzugsweise 0,001 bis 5 Gew.-%, bezogen auf das Gesamtgewicht der Zusammensetzung, liegt.

11. Zusammensetzung nach einem der Ansprüche 1 bis 10, die ferner mindestens ein mono- oder polycarboxyhaltiges Selbstbräunungsmittel enthält.

12. Zusammensetzung nach Anspruch 11, wobei das mono- oder polycarboxyhaltige Selbstbräunungsmittel oder die mono- oder carboxyhaltigen Selbstbräunungsmittel unter Isatin, Alloxan, Ninhydrin, Glycerinaldehyd, Mesoweinsäurealdehyd, Glutaraldehyd, Erythrulose, Pyrazolin-4,5-dionderivaten, Dihydroxyaceton (DHA) und 4,4-Dihydroxypyrazolin-5-onderivaten ausgewählt sind, wobei diese Selbstbräunungsmittel gegebenenfalls mit Direktfarbstoffen oder Indolderivaten kombiniert werden können.

13. Zusammensetzung nach Anspruch 11 oder 12, wobei das Selbstbräunungsmittel das Dihydroxyaceton (DHA) ist.

14. Zusammensetzung nach einem der Ansprüche 11 bis 13, wobei das Selbstbräunungsmittel in Anteilen von 0,1 bis 10 Gew.-%, bezogen auf das Gesamtgewicht der Zusammensetzung, vorliegt.

15. Zusammensetzung nach einem der Ansprüche 1 bis 14, die ferner mindestens ein UV-Filter enthält.

16. Zusammensetzung nach Anspruch 15, wobei das UV-Filter unter den organischen UV-Filtern oder anorganischen Stoffen, die UV-Strahlung filtern, ausgewählt ist.

17. Zusammensetzung nach Anspruch 16, wobei das oder die organischen UV-Filter wasserlöslich, fettlöslich oder in üblichen kosmetischen Lösungsmitteln unlöslich sind.

18. Zusammensetzung nach Anspruch 17, wobei das oder die organischen UV-Filter unter Anthranilaten; Zimtsäurederivaten; Dibenzoylmethanderivaten; Salicylsäurederivaten; Campherderivaten; Triazinderivaten; Benzophenonderivaten; β, β'-Diphenylacrylatderivaten, Benzotriazolderivaten, Benzimidazolderivaten; Imidazolinen; Bis-benzoazolylderivaten; p-Aminobenzoesäurederivaten (PABA); Methylen-bis(hydroxyphenylbenzotriazol)-derivaten; Polymerfiltern und Siliconfiltern; von α-Alkylstyrol abgeleiteten Dimeren; 4,4-Diarylbutadienderivaten und deren Gemischen ausgewählt sind.

**19.** Zusammensetzung nach Anspruch 18, wobei das oder die organischen UV-Filter ausgewählt sind unter:

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Benzimidazilate,
- Anisotriazine,
- Ethylhexyl Triazone,
- Diethylhexyl Butamino Triazone,
- 2,4,6-Tris-(diisobutyl-4'-amino-benzalmalonat)-2-triazin,
- Methylene-bis-benzotriazolyl-tetramethylbutylphenol,
- Drometrizole Trisiloxane,
- 1, 1-Dicarboxy-(2,2'-dimethyl-propyl)-4,4-diphenylbutadien,

und deren Gemischen.

**20.** Zusammensetzung nach Anspruch 16, wobei das oder die anorganischen UV-Filter unter den Pigmenten oder Nanopigmenten von Metalloxiden, die umhüllt oder nicht umhüllt sind, ausgewählt sind.

**21.** Zusammensetzung nach Anspruch 20, wobei das oder die anorganischen UV-Filter unter den Nanopigmenten von Titanoxid, Eisenoxid, Zinkoxid, Zirconiumoxid oder Ceroxid, die umhüllt oder nicht umhüllt sind, ausgewählt sind.

**22.** Zusammensetzung nach einem der Ansprüche 15 bis 21, wobei das oder die UV-Filter in Mengenanteilen von 0,1 bis 15 Gew.-%, bezogen auf das Gesamtgewicht der Zusammensetzung, enthalten sind.

**23.** Verfahren zur künstlichen Färbung der Haut, die der natürlichen Bräunung nahe kommt, **dadurch gekennzeichnet, dass** es darin besteht, auf die Haut eine wirksame Menge einer Zusammensetzung nach einem der Ansprüche 1 bis 10 aufzutragen.

**24.** Verwendung mindestens eines Sorghum-Extrakts, wie er in den vorhergehenden Ansprüchen definiert wurde, in einer kosmetischen Zusammensetzung, die kein in 3-Stellung unsubstituiertes und mit mindestens einer Hydroxy- oder Alkoxygruppe substituiertes Flavyliumsalz enthält, um der Haut eine künstliche Färbung zu geben, die der natürlichen Bräunung nahe kommt.